# EUROPEAN PATENT SPECIFICATION

(11) **EP 4 426 361 B1**
(45) Date of publication and mention of the grant of the patent: **28.01.2026**
(21) Application number: 22706413.6
(22) Date of filing: 16.02.2022
(51) Int. Cl.: A61L 2/24, F24F 3/16, G06Q 50/00, G16H 40/00

(54) **DISINFECTION OF SURFACES IN CONFINED ENVIRONMENTS**
DESINFEKTION VON OBERFLÄCHEN IN GESCHLOSSENEN UMGEBUNGEN
DÉSINFECTION DE SURFACES DANS DES ENVIRONNEMENTS CONFINÉS

(30) Priority: 21.10.2021 IT 202100027041
(43) Date of publication of application: 11.09.2024
(73) Proprietor: Work In Progress Bio-medical S.r.l., 20129 Milano (IT)
(72) Inventor: LONGO, Matteo, 10064 Pinerolo (Torino) (IT)
(74) Representative: Fioravanti, Corrado
(86) International application number: PCT/IB2022/051378
(87) International publication number: WO 2022/224047

(56) References cited:
- EP-B1- 3 356 742
- WO-A2-2012/033850
- US-A1- 2015 205 985

## Description

### Technical field

The present invention refers to a method for performing, tracking and controlling disinfection of a confined environment and of the objects contained therein.

### Background art

In the field of sanitation, disinfection of surfaces in confined spaces is conventionally performed following pre-established operating procedures manually performed by an operator, which include applying chemical disinfectant products on surfaces of confined spaces, rinsing and drying the surfaces. The traceability of disinfection activities of the surfaces in confined spaces is carried out manually by an operator. In particular, the operator manually notes in a special register both the frequency and the results of the disinfection procedures carried out on the confined environment.

The traceability of the disinfection carried out manually by the operator may be subject to errors both in transcribing information regarding the disinfection procedure in the register, and in evaluating the validity of the disinfection procedure that has been carried out. Such registration and evaluation errors make the manual traceability method unreliable. Furthermore, errors in the traceability of disinfection may also have consequences on the health of the operator and on safety of the activities to be carried out within the confined environment after the disinfection procedures.

To overcome these drawbacks, a few recently proposed solutions suggest performing disinfection procedures of confined spaces by nebulization devices.

WO 2014/006577 A1 discloses a system for reducing bacterial content in a confined environment, comprising a nebulization device that delivers a decontaminating agent for a time calculated on the basis of information including the volume of the confined environment in which it is contained and the technical specifications of the decontaminating agent to be dispensed. Furthermore, the system described in WO 2014/006577 A1 is able to assess the concentration of the decontaminating agent in the confined environment and, therefore, to certify the conformity or non-conformity of the disinfection procedure of the confined environment.

Document US 2015/205985 A1 describes a method for arranging the disinfection of one or more objects contaminated with biological agents comprising attaching a radio frequency identification label to the object to be disinfected, exposing the object to a disinfection medium for a period sufficient to disinfect the object, and obtaining a signal from the labeled object when the disinfection is completed. Document US 2015/205985 A1 further describes a method of disinfecting one or more objects contaminated with biological agents, comprising the step of exposing the object to be disinfected to a disinfection medium for a period sufficient to disinfect the object, wherein the object has a radio frequency identification label, and obtaining a signal from the radio frequency labelled object when disinfection is completed.

Document WO 2012/033850 A2 describes a device that atomises a biocidal agent in the form of a dry mist and, after a certain period of time, withdraws the dry mist or biocidal agent from the surrounding environment. Each device can implement tracking or monitoring of operational parameters. To track or monitor the device's operational parameters, a separate tracking or monitoring system may also be provided. Tracking or monitoring may include exception reporting and other alerts.

### Summary of the invention

A general object of the present invention is to guarantee bacterial decontamination of confined environments including the surfaces of objects and equipment, and to prevent or at least limit the bacterial diffusion and contamination of already sanitized environments, especially in structures with a plurality of environments to keep sanitized.

A particular object of the present invention is to improve the efficiency of the traceability of disinfection procedures.

According to another aspect, the present invention aims at reducing waste of the decontaminating agent used for the sanitization of confined environments.

These and other objects and advantages, which will be better understood hereinafter, are achieved according to the present invention by a method and a system having the features defined in the appended claims.

In summary, the present invention is based on a method for carrying out, tracing and controlling airborne disinfection of the surfaces of a confined environment and the objects contained therein. The proposed method includes the steps of:
- associating to a confined environment an environmental identification device configured to contain identifying information about the confined environment and its volume;
- providing in the confined environment a plurality of objects, each equipped with an associated recognition means containing information identifying the object and information relating to the volume of the associated object;
- providing a sanitizing apparatus in the confined environment to dispense airborne decontaminating agents,
- acquiring identification information about the confined environment from the environmental identification device, acquiring information from said recognition means associated with the objects, acquiring information about a decontaminating agent to be dispensed into the confined environment, and processing said acquired information by control and processing means;
- calculating a volume of air contained in the confined environment by subtracting from the volume of the confined environment the volume occupied by the objects contained therein;
- calculating a dispensing time based on the type of decontaminating agent being dispensed and on the volume of air contained in the confined environment;
- dispensing said decontaminating agent in the air by a diffuser element of the sanitizing apparatus for the dispensing time calculated by the control and processing means on the basis of the acquired information;
- detecting the concentration of airborne decontaminating agent in the confined environment; and
- providing certification information, depending on the detected concentration of decontaminating agent, certifying that the surfaces of the confined environment and the objects contained therein and associated with the recognition means have been disinfected during the dispensing step.

The expected qualitative result (disinfection, high disinfection or sterilization) is guaranteed by monitoring the concentration of the airborne product. As better explained hereinafter, the method allows documenting that the contact time between the airborne product and the surfaces contained in the confined environment, at a set concentration, has taken place in compliance with a predefined quality standard. Usually, reference is made to the EN standards followed in the experimental / registration phase. Furthermore, the method guarantees that the time for restoring the minimum safety conditions has been checked.

### Brief description of the drawings

The features and advantages of the present invention will be better understood from the detailed description of some embodiments thereof, provided by way of non-limiting example with reference to the attached drawings, in which:
Figure 1 is a perspective view of a confined environment in which there are an environmental identification device, a plurality of objects, and a sanitizing apparatus;
Figure 2 is a front view of an environmental identification device comprising memory means;
Figure 3 is a perspective view of a sanitizing apparatus for dispensing decontaminating agents;
Figure 4A is a perspective view of an identification sensor according to an embodiment of the present invention;
Figure 4B is a perspective view of a recognition means according to an embodiment of the present invention; and
Figure 5 represents a graph showing a trend of a decontaminating agent concentration during the disinfection process.

### Detailed description

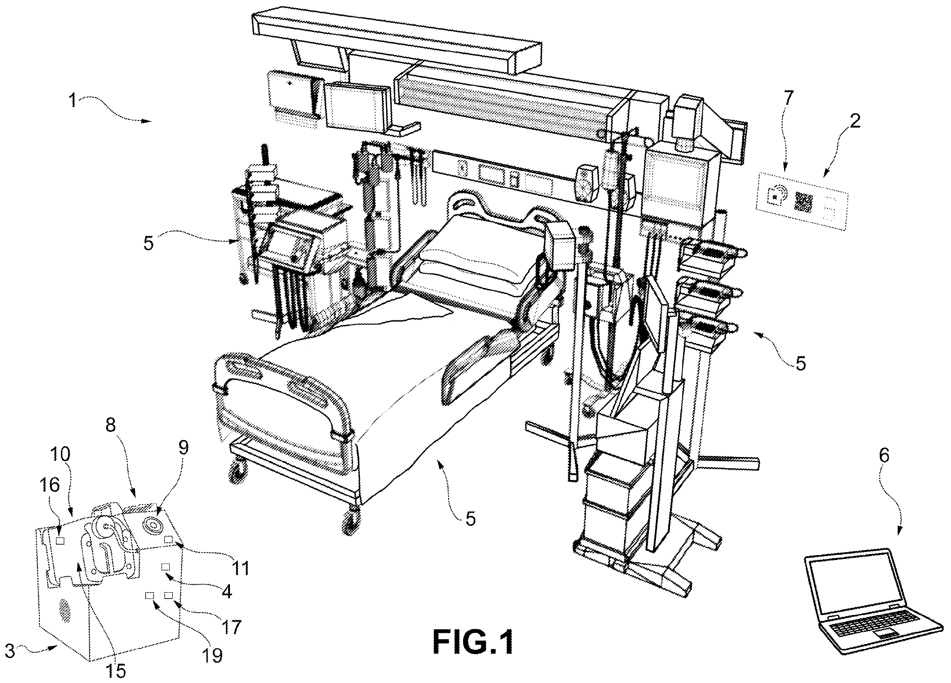

Referring initially to Figure 1, an embodiment of a system for performing, tracking, monitoring and controlling disinfection by air of surfaces of a confined environment 1 and objects contained therein is first described.

Typically, the confined environment 1 may be a confined hospital environment, such as an operating room. The confined environment 1 may also be a confined industrial environment, and/or a confined public environment, such as an office, and/or a confined private environment, such as a domestic environment.

The system comprises at least one environmental identification device 2, an identification device 16 of a decontaminating agent 10, a plurality of recognition means 5, a sanitizing apparatus 3, first identification sensor means 4, second sensor means 8, a detection sensor 11, and control and processing means 17, 19.

The environmental identification device 2 is located inside the confined environment 1, and may be placed in a fixed and/or removable manner. The environmental identification device 2 is configured to contain and make available information that uniquely identifies the confined environment 1 and information related to the volume of the confined environment 1.

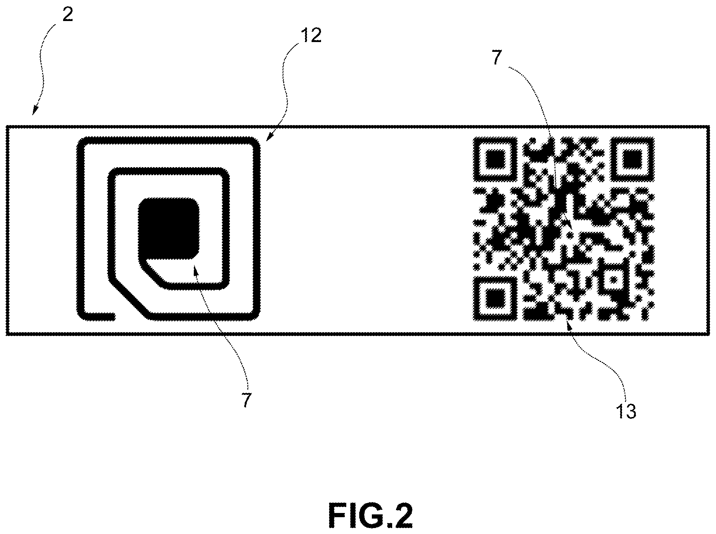

The environmental identification device 2 may be internally equipped with memory means 7, known per se, inside which there may be a writable printed circuit 12, or an identification QR Code 13 (Figure 2).

In a preferred embodiment, the information contained in the environmental identification device 2 may be information regarding the architecture of the confined environment 1.

In addition, the environmental identification device 2 may contain information about frequency of execution of the decontamination treatment carried out in the confined environment 1.

According to an embodiment, the identification device 16 of the decontaminating agent 10 may be located on a collection tank 15 of the decontaminating agent 10.

The identification device 16 of the decontaminating agent 10 may include a label inside which there may be a writable printed circuit 12, or an identification QR Code 13.

Information about the decontaminating agent 10 contained in the identification device 16 comprises the type of decontaminating agent to be diffused, and/or may comprise information related to its optimal dispensing concentration.

The confined environment 1 contains a plurality of obj ects, each of which is associated with an individual recognition means 5.

The objects contained within the confined environment 1 may be fixed in the confined environment 1 and/or movable, therefore transportable from a confined environment to another confined environment.

In applications where the confined environment 1 is part of a hospital environment, the objects contained in the confined environment 1 may be, by way of example but not limited to, medical devices, such as a bed, a defibrillator, an electrocardiograph and the like.

Each recognition means 5 associated with an object contained in the confined environment 1 contains and provides identifying information about the object to which it is associated and information relating to the volume of the associated object.

Each recognition means 5 may include a writable printed circuit 12 and/or an identification QR Code 13.

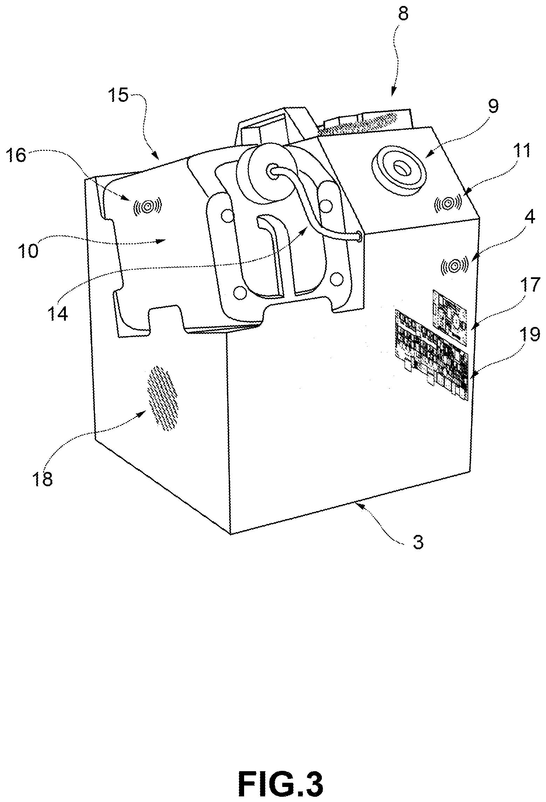

The sanitizing apparatus 3 is equipped with a diffuser 9 for dispensing airborne decontaminating agents 10 in confined environments 1 (Figure 3).

The specific constructional and functional features of the mechanical parts of the sanitizing apparatus 3 (which may be, for example, those disclosed in WO 2014/006577 A1,) are not relevant for understanding the present invention; therefore, they will not be described in detail herein. The exemplary sanitizing apparatus illustrated in Figure 3 comprises a tank 15 configured to contain the decontaminating agent 10, a feeding cannula 14 configured to collect the decontaminating agent 10 from the tank 15, and an electric motor 18 associated with a fan (not shown). Due to a Venturi effect, the fan collects the decontaminating agent from the tank 15 through the cannula 14 and sucks in air from the confined environment 1, mixes the decontaminating agent proportionally to the sucked air and emits into the environment, through the diffuser 9, a mixture of air and nebulized decontaminating agent.

Preferably, the diffuser 9 is provided with a diffuser nozzle made to mix, according to a predetermined proportion, air and decontaminating agents 10 in the form of dry fog.

According to an embodiment, the sanitizing apparatus 3 may be equipped with a diffuser nozzle associated with a system for calibrating the quantity of decontaminating agent 10 which is sucked in by the electric blower motor 18 and transformed into dry fog through a number of inclined ducting holes at the outlet of the mixed air, generating a centrifugal turbulence that facilitates the dispersion in the environment of particles with dimensions between 2 and 10 microns, preferably 5 microns, avoiding their deposit on the surfaces and creation of humidity.

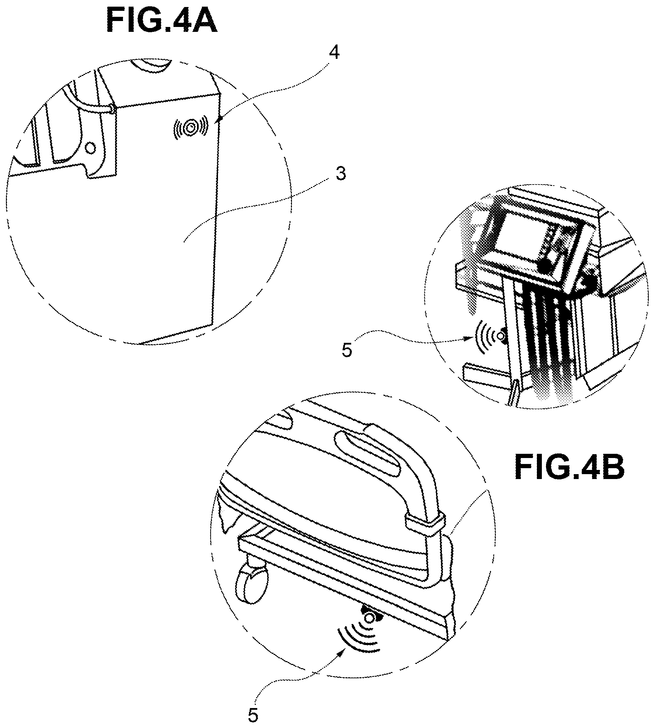

The first identification sensor means 4 (Figure 4A) are configured to acquire the identification information about the objects located within the confined environment 1, contained in the recognition means 5 (Figure 4B) associated with these objects.

In certain embodiments the first identification sensor means 4 may modulate the width of the search range for objects as a function of the volumetric dimension of the confined environment 1 to be sanitized in which the sanitizing apparatus 3 is placed each time. The search range of the identification sensors means 4 may increase or decrease according to the acquired data relating to the volume of the confined environment, based on the data made available by the environmental identification device 2.

The second sensor means 8 are configured to acquire the identifying information about the confined environment 1 contained in the environmental identification device 2 and to acquire the identifying information about the decontaminating agent 10, to be diffused in the confined environment 1, contained in the identification device 16 of the decontaminating agent 10.

In an embodiment, the second sensor means 8 may comprise a transponder.

The first sensor means 4 and the second sensor means 8 may comprise sensors configured for reading labels with RFID technology, and/or QR code.

The first sensor means 4, the second sensor means 8, the detection sensor 11 for detecting the concentration of decontaminating agent 10, and the control and processing means 17, 19 may be mounted on the sanitizing apparatus 3.

In certain embodiments the first sensor means and the second sensor means coincide, so that a single sensor or reader (for example, an RFID sensor) may be used both to identify the objects 5 and acquire the identifying information about the confined environment 1.

The detection sensor 11 is configured to detect the concentration of decontaminating agent 10 air-dispersed from the diffuser 9 of the sanitizing apparatus 3 inside the confined environment 1.

The control and processing means 17, 19 are configured to store and process the identifying information about the objects contained in the confined environment 1 acquired by the first sensor means 4, the information of the confined environment 1 and the identifying information of the decontaminating agent 10 acquired by the second sensor means 8, and the information on the concentration of air-dispersed decontaminating agent in the confined environment 1 acquired by the detection sensor 11.

In addition, the control and processing means 17, 19 may be configured to supervise operation and/or coordinate the activity of the sanitizing apparatus 3 as a whole, and, in particular, to supervise operation and/or control the activity of the diffuser 9 and all associated devices for its startup, operation, and shutdown.

The control and processing means 17, 19 are configured to make certification information available and certify whether the objects contained in the confined environment have been disinfected or not, according to the concentration of decontaminating agent 10 detected by the detection sensor 11.

The system may be associated, in a per se known manner, to a central server 6 configured to coordinate the activity of the various devices of the system.

The central server 6 may be configured to receive information about the disinfection process of the surfaces of the confined environment 1 and of the objects contained therein, and, in particular, the certification information made available by the control and processing means 17, 19 on compliance or noncompliance of the disinfection of the surfaces of the confined environment 1 and of the objects contained therein during the dispensing of the decontaminating agent 10.

The certification information may be transmitted to the central server 6, for example via Bluetooth, sms, gprs and other telecommunication technologies, or via cable. As an alternative, the certification information may be loaded from the sanitizing apparatus 3 onto a removable memory medium such as a USB key, and then transmitted to the server.

**In** certain embodiments, a transponder 8, in addition to acting as a second sensor means for acquiring identification information about the confined environment, is configured to transmit the certification information processed by the control and processing means 17, 19 to the central server 6.

Data acquired by the central server 6 may be processed by the central server 6 itself to highlight anomalies with respect to the expected objective and certify the disinfection activities that have been performed and their history, in accordance with a pre-established control plan and with the creation of an "operational technical dossier" defined for each individual disinfected object 5 (for example, a medical device) and for each disinfected confined environment.

The steps of a method for performing, tracking and controlling airborne disinfection of surfaces of a confined environment 1 and of the objects contained therein will now be described.

First, the environmental identification device 2 configured to contain identifying information about the confined environment 1 and its volume is provided in the confined environment 1 or in its proximity. A plurality of objects is arranged in the confined environment 1, each object being equipped with an associated means of recognition 5 which contains identifying information about the object and information relating to its volume. Then, the sanitizing apparatus 3, configured to dispense the airborne decontaminating agent 10, is placed in the confined environment. Identification information about the confined environment 1 contained in the environmental identification device 2 is then acquired by the second sensor means 8; identification information about the objects is acquired from the recognition means 5 associated with the objects by the first sensor means 4 and identification information about the decontaminating agent 10 to be diffused in the confined environment 1 is acquired from the identification means 16 of the decontaminating agent 10 by the second sensor means 8. Finally, acquired information is processed by the control and processing means 17, 19.

Then a volume of air contained in the confined space 1 is calculated by subtracting the volume occupied by the objects contained therein from the volume of the confined space. Then calculate a dispensing time based on the type of decontamination agent 10 to be dispensed and the volume of air contained in the confined space.

In other words, the sanitizing apparatus 3 first identifies the environment in which it is positioned. Then, it identifies the quantity of objects present inside the room to determine air volume in the room and, based on this, sets the dispensing time of the decontaminating agent 10. It subtracts the volume of the identified objects to the volume of the environment, resulting in the volume of air to be treated.

Subsequently, the decontaminating agent 10 is airborne delivered by the diffuser 9 of the sanitizing apparatus 3 for the dispensing time calculated by the control and processing means 17, 19 based on acquired information. The concentration of decontaminating agent 10 air-dispersed in the confined environment 1 is detected by the detection sensor 11.

Finally, according to the concentration of decontaminating agent detected in the confined environment 1 by the detection sensor 11, the control and processing means 17, 19 provide certification information, which certifies disinfection of the surfaces of the confined environment 1 and of the objects contained therein and associated with the recognition means 5 during dispensing of the decontaminating agent 10.

Preferably, the sanitizing apparatus 3 defines the preliminary dose of decontaminating agent 10 to be dispensed in order to achieve the presumed optimal concentration of decontaminating agent 10 air-dispersed in the confined environment 1.

Checking of compliance of the treatment takes place through the detection sensor 11 of the airborne product, operationally associated with the control and processing means 17, 19, which coordinate activation and deactivation of the decontaminating agent dispensing device.

In a preferred embodiment, the concentration of decontaminating agent 10 air-dispersed in the confined environment 1 may be periodically or continuously monitored during dispensing by the detection sensor 11.

In certain embodiments method comprises modulating the dispensing step, regulating switching off and on of the sanitizing apparatus 3 so that the air-dispersed decontaminating agent remains in contact with the surfaces to be sanitized for an overall prescribed duration (or contact time hereinafter), with concentration values not lower than a predetermined minimum threshold concentration value.

According to an embodiment, the apparatus 3 is configured to extend the dispensing time of the decontaminating agent if, during the dispensing step, the detection sensor 11 signals that the concentration of air-dispersed decontaminating agent is lower than a predetermined minimum threshold concentration value.

As an alternative, or in addition, according to another embodiment, the apparatus 3 is configured to extend the dispensing time of decontaminating agent by switching on again to resume dispensing of decontaminating agent if, once the dispensing time calculated by the control and processing means 17, 19, and once the dispensing has ceased, data made available by the detection sensor 11 indicate that during the previous dispensing step, the concentration of air-dispersed decontaminating agent was lower than a predetermined concentration value minimum threshold. Alternative embodiments comprise verifying achievement of the concentration of decontaminating agent once the calculated dispensing time has elapsed.

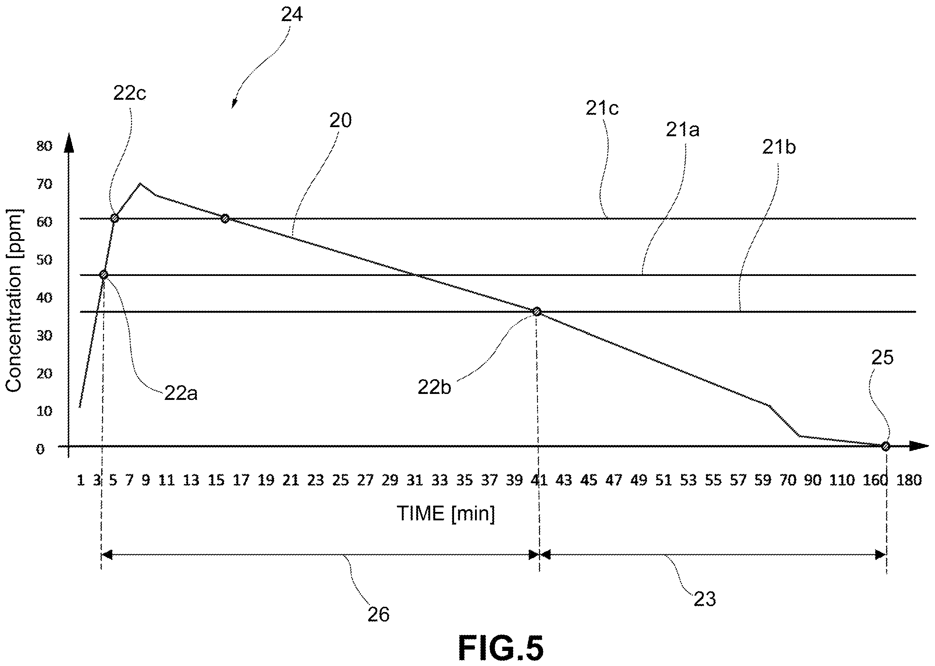

Certification that disinfection of the surfaces of the confined environment 1 and of the objects contained therein has taken place may include a graph 24 (figure 5) which shows a curve 20 representing variation over time of the concentration of air-dispersed decontaminating agent in the confined environment 1 during the disinfection procedure.

The graph indicates an optimal concentration value 21a, a minimum concentration value 21b, and a maximum concentration value 21c, defined according to the decontaminating agent 10 delivered during the disinfection.

In addition, the graph 24 indicates a starting time 22a, in which the concentration of decontaminating agent 10 air-dispersed in the confined environment 1 reaches the optimal concentration value 21a.

The graph 24 indicates a peak time 22c, in which the concentration of decontaminating agent 10 air-dispersed in the confined environment 1 reaches the maximum concentration value 21c. At this time instant, the apparatus 3 stops dispensing decontaminating agent 10.

In addition, the graph 24 indicates an ending time 22b, in which the concentration of decontaminating agent 10 air-dispersed in the confined environment 1 reaches the minimum concentration value 21b.

Preferably, the graph 24 further indicates contact time 26 of the surfaces of the confined environment 1 and of the objects contained therein with the decontaminating agent 10. The contact time 26 may be calculated as the difference between the ending time 22b and starting time 22a.

The graph 24 may further indicate a safety time 25, in which the concentration of decontaminating agent 10 air-dispersed in the confined environment 1 reaches the zero-concentration value, and/or restoration of environmental safety conditions.

Finally, the graph 24 may indicate a decay time 23 of the concentration of air-dispersed decontaminating agent. The decay time 23 may be calculated as the difference between the safety time 25 and the ending time 22b.

According to an embodiment, the method of the present invention comprises checking that the contact time of the decontaminating agent used and concentrated in the environment is maintained for a time necessary to guarantee the effectiveness certified in a previous testing and validation phase of the chemical product at the time of its official registration. **In** order to declare, for example, that a chemical product is a disinfectant, it is known that it is necessary to demonstrate how much of chemical product must be used (concentration) and how long it must remain in contact with the surface (contact time) to demonstrate its bactericidal ability.

For an optimal implementation of the method, the contact time 26, determined by the interval between the instants 22a and 22b, must be monitored and maintained for a predetermined duration.

The contact time 26 begins to run, during the dispensing step, at the instant in which the level 21a (optimal concentration value) is reached, and continues with reaching and exceeding the level 21c (maximum concentration value), which coincides with stopping dispensing (according to a dispensing time determined on the basis of the calculated air volume). The contact time 26 lasts until the level 21b (minimum concentration value) is reached. By way of example, with the dispensing of an active ingredient based on hydrogen peroxide H2O2, the contact time may be 20 minutes. Therefore, it is not sufficient that the detected decontaminant concentration reaches or exceeds a certain value, but the concentration must not fall below a minimum threshold concentration value (21b) for a predetermined duration (contact time 26).

In the example of figure 5, the graph 24 refers to a monitoring phase of the disinfection method performed in a confined sanitary environment 1, in which an active disinfectant based on hydrogen peroxide at a concentration of 12% is airborne. The graph highlights:
- the saturation path of the concentration in parts per million until reaching the maximum concentration threshold 21c, normally between 40 ppm and 80 ppm, even more preferably in the amount of 60 ppm;
- the overall contact time 26 necessary to ensure the broad spectrum of action on viruses, fungi, bacteria and spores detected with instants 22a and 22b is between 10 and 30 minutes, even more preferably 20 minutes;
- the decay or degradation time 23, normally comprised between 30 and 180 minutes, even more preferably 60 minutes, suitable for restoring the minimum safety conditions 25 below 2 ppm of airborne chemical product 10.

## Claims

1. A method of performing, tracking, and controlling airborne disinfection of surfaces of a confined environment (1) and objects contained therein, the method comprising the steps of:
- associating to a confined environment (1) an environmental identification device (2) configured to contain identifying information about the confined environment (1) and its volume;
- providing in the confined environment (1) a plurality of objects, each equipped with an associated recognition means (5) containing information identifying the object and information relating to the volume of the associated object;
- providing a sanitizing apparatus (3) in the confined environment to dispense airborne decontaminating agents;
- acquiring identification information about the confined environment (1) from the environmental identification device (2), acquiring information from said recognition means (5) associated with the objects, acquiring information about a decontaminating agent (10) to be dispensed into the confined environment, and processing said acquired information by control and processing means (17, 19);
- calculating a volume of air contained in the confined environment (1) by subtracting from the volume of the confined environment the volume occupied by the objects contained therein;
- calculating a dispensing time based on the type of decontaminating agent (10) being dispensed and on the volume of air contained in the confined environment;
- dispensing said decontaminating agent (10) in the air by means of a diffuser element (9) of the sanitizing apparatus (3) for the dispensing time calculated by the control and processing means (17, 19) on the basis of the acquired information;
- detecting the concentration of airborne decontaminating agent (10) in the confined environment (1); and
- providing certification information, depending on the detected concentration of decontaminating agent, certifying that the surfaces of the confined environment and the objects contained therein and associated with the recognition means (5) have been disinfected during the decontaminating agent dispensing step.

2. A method according to claim 1, wherein the concentration of airborne decontaminating agent in the confined environment (1) is monitored during the dispensing of said agent.

3. A method according to claims 1 or 2, wherein the delivery of the decontaminating agent (10) is controlled such that the concentration of said airborne agent (10) in the confined environment (1) does not decrease below a predetermined concentration value for a predetermined period (26).

4. A method according to any one of the preceding claims, wherein the dispensing of the decontaminating agent (10) is prolonged if, during the dispensing step, the detected concentration of the aero-dispersed decontaminating agent is below a predetermined concentration value.

5. A method according to any one of the preceding claims, wherein the dispensing of decontaminating agent (10) is restarted if, after said dispensing time calculated by the control and processing means (17, 19) has elapsed and the dispensing has ceased, the detected concentration of airborne decontaminating agent has been below a predetermined concentration value during the preceding dispensing step.

6. A system for performing, tracking, monitoring and controlling airborne disinfection of surfaces of a confined environment (1) and objects contained therein, the system comprising:
- at least one environmental identification device (2), located in a confined environment (1), the identification device being configured to contain and provide identification information about the confined environment (1) and information relating to the volume of the confined environment (1);
- at least one identification device (16) of the decontaminating agent (10) configured to contain and provide identification information about the decontaminating agent (10);
- a plurality of recognition means (5), each of which associated with an object located in the confined environment (1), wherein each recognition means (5) contains identification information about the associated object and information relating to the volume of the associated object;
- a sanitizing apparatus (3) equipped with a diffuser (9) for the airborne delivery of decontaminating agents (10);
- first sensors means of identification (4) for acquiring information contained in the recognition means (5) associated with the objects located within the confined environment;
- second sensors means (8) adapted for acquiring identification information about the confined environment (1) from the environmental identification device (2), and adapted for acquiring identification information about the decontaminating agent (10) to be dispensed in the confined environment (1);
- a detection sensor (11) adapted to detect the concentration of the decontaminating agent (10) airborne within the confined environment (1);
- control and processing means (17, 19) for storing and processing the information acquired by said sensors and providing certification information, depending on the detected concentration of decontaminating agent, on the compliance or noncompliance of the disinfection of the surfaces of the confined environment and the objects contained therein during the step of dispensing the decontaminating agent.

7. A system according to claim 6, wherein said first and second sensor means comprise sensors configured to read tags using either RFID or QR code technology.

8. A system according to claim 6 or 7, wherein said second sensors means (8) comprise a transponder.

9. A system according to any one of claims 6 to 8, wherein the sanitizing apparatus (3) comprises: said first (4) and second (8) sensors means, the detection sensor (11) the concentration of decontaminating agent, the control and processing means (17, 19).

## Patentansprüche

1. Verfahren zur Durchführung, Verfolgung und Steuerung von luftgetragenen Desinfektion von Oberflächen einer geschlossenen Umgebung (1) und von Objekten, die enthalten werden, das Verfahren umfassend die folgenden Schritte:
- Zuordnen einer geschlossenen Umgebung (1) eine Umgebungsidentifikationsvorrichtung (2), die dazu konfiguriert ist, Identifikationsinformationen über die geschlossene Umgebung (1) und deren Volumen zu enthalten;
- Vorsehen in der geschlossenen Umgebung (1) einer Mehrzahl von Objekten, wobei jedes mit einem zugeordneten Erkennungsmittel (5) ausgestattet ist, das Informationen zur Identifikation des Objekts sowie Informationen bezogen auf das Volumen des zugeordneten Objekts enthält;
- Vorsehen einer Hygienisierungsvorrichtung (3) in der geschlossenen Umgebung zum Abgeben luftgetragener Dekontaminationsmittel;
- Erfassen Identifikationsinformationen über die geschlossene Umgebung (1) von der Umgebungsidentifikationsvorrichtung (2), Erfassen Informationen aus dem Erkennungsmittel (5), das den Objekten zugeordnet ist, Erfassen Informationen über ein Dekontaminationsmittel (10), das in die geschlossene Umgebung ausgegeben werden soll und Verarbeiten der erfassten Informationen durch Steuerung- und Verarbeitungsmittel (17, 19);
- Berechnen eines Volumens von Luft, die in der geschlossenen Umgebung (1) enthalten ist, indem das Volumen, das durch die darin enthaltenen Objekten belegt wird, aus dem Volumen der geschlossenen Umgebung abgezogen wird;
- Berechnen einer Ausgabezeit basierend auf dem Typ des auszugebenden Dekontaminationsmittels (10) und dem Volumen von Luft, die in der geschlossenen Umgebung enthalten ist;
- Ausgeben des Dekontaminationsmittels (10) in die Luft mittels eines Diffusorelements (9) der Hygienisierungsvorrichtung (3) für die Ausgabezeit, die durch die Steuerung- und Verarbeitungsmittel (17, 19) basierend auf der erfassten Informationen berechnet wird;
- Erfassen der Konzentration des luftgetragenen Dekontaminationsmittels (10) in der geschlossenen Umgebung (1); und
- Bereitstellen Zertifizierungsinformationen, in Abhängigkeit von der erfassten Konzentration des Dekontaminationsmittels, die bestätigen, dass die Oberflächen der geschlossenen Umgebung sowie die Objekte, die darin enthalten sind und mit den Erkennungsmitteln (5) zugeordnet sind, während des Dekontaminationsmittel-Ausgabeschritt desinfiziert wurden.

2. Verfahren nach Anspruch 1, wobei die Konzentration des luftgetragenen Dekontaminationsmittels in der geschlossenen Umgebung (1) während der Ausgabe des Mittels überwacht wird.

3. Verfahren nach Anspruch 1 oder 2, wobei die Ausgabe des Dekontaminationsmittels (10) gesteuert wird, sodass die Konzentration des luftgetragenen Mittels (10) in der geschlossenen Umgebung (1) über einen vorbestimmten Zeitraum (26) nicht unter einen vorbestimmten Konzentrationswert absinkt.

4. Verfahren nach einem der vorhergehenden Ansprüche, wobei die Ausgabe des Dekontaminationsmittels (10) verlängert wird, wenn während des Ausgabevorgangs die erkannte Konzentration des luftdispergierten Dekontaminationsmittels unter einem vorbestimmten Konzentrationswert liegt.

5. Verfahren nach einem der vorhergehenden Ansprüche, wobei die Ausgabe des Dekontaminationsmittels (10) erneut gestartet wird, wenn nachdem die Ausgabezeit, die durch die Steuerung- und Verarbeitungsmittel (17, 19) berechnet wird, abgelaufen ist und nachdem die Ausgabe beendet ist, die erkannte Konzentration des luftgetragenen Dekontaminationsmittels während des vorherigen Ausgabevorgangs unter einem vorbestimmten Konzentrationswert lag.

6. System zur Durchführung, Verfolgung, Überwachung und Steuerung der luftgetragenen Desinfektion von Oberflächen einer geschlossenen Umgebung (1) und Objekten, die darin enthalten sind, das System umfassend:
- mindestens eine Umgebungsidentifikationsvorrichtung (2), die sich in einer geschlossenen Umgebung (1) befindet, wobei die Identifikationsvorrichtung dazu konfiguriert ist, Identifikationsinformationen über die geschlossene Umgebung (1) sowie Informationen bezogen auf das Volumen der geschlossenen Umgebung (1) zu enthalten und bereitzustellen;
- mindestens eine Identifikationsvorrichtung (16) des Dekontaminationsmittels (10), die dazu konfiguriert ist, Identifikationsinformationen über das Dekontaminationsmittel (10) zu enthalten und bereitzustellen;
- eine Mehrzahl von Erkennungsmitteln (5), von denen jedes einem Objekt, das sich in der geschlossenen Umgebung (1) befindet, zugeordnet ist, wobei jedes Erkennungsmittel (5) Identifikationsinformationen über das zugeordnete Objekt sowie Informationen bezogen auf das Volumen des zugeordneten Objekts enthält;
- eine Hygienisierungsvorrichtung (3), die mit einem Diffusor (9) für die luftgetragene Ausgabe der Dekontaminationsmittel (10) ausgestattet ist;
- erste Identifikation-Sensormittel (4) zum Erfassen Informationen, die in den Erkennungsmitteln (5) enthalten sind, die den Objekten zugeordnet sind, die sich in der geschlossenen Umgebung befinden;
- zweite Sensormittel (8), die eingerichtet sind, Identifikationsinformationen über die geschlossene Umgebung (1) von der Umgebungsidentifikationsvorrichtung (2) zu erfassen und die eingerichtet sind, Identifikationsinformationen über das Dekontaminationsmittel (10), das in der geschlossenen Umgebung (1) ausgegeben werden soll, zu erfassen;
- einen Erkennungssensor (11), der eingerichtet ist, die Konzentration des luftgetragenen Dekontaminationsmittels (10) innerhalb der geschlossenen Umgebung (1) zu erfassen;
- Steuerungs- und Verarbeitungsmittel (17, 19) zum Speichern und Verarbeiten der Informationen, die durch die Sensoren erfasst werden und zum Bereitstellen Zertifizierungsinformationen, in Abhängigkeit von der erkannten Konzentration des Dekontaminationsmittels, über die Konformität oder Nichtkonformität der Desinfektion der Oberflächen der geschlossenen Umgebung und der Objekte, die darin enthalten sind, während des Schritts von Ausgeben des Dekontaminationsmittels.

7. System nach Anspruch 6, wobei die ersten und zweiten Sensormittel Sensoren umfassen, die dazu konfiguriert sind, Tag durch Verwenden von RFID oder QR Code-Technologie zu lesen.

8. System nach Anspruch 6 oder 7, wobei die zweiten Sensormittel (8) einen Transponder umfassen.

9. System nach einem der Ansprüche 6 bis 8, wobei die Hygienisierungsvorrichtung (3) umfasst: erste (4) und zweite (8) Sensormittel, den Erkennungssensor (11) der Konzentration des Dekontaminationsmittels sowie die Steuerung- und Verarbeitungsmittel (17, 19).

## Revendications

1. Procédé de réalisation, de suivi et de contrôle de la désinfection par voie aérienne des surfaces d'un environnement confiné (1) et des objets qui y sont contenus, ledit procédé comprenant les étapes consistant à :
associer à un environnement confiné (1) un dispositif d'identification environnementale (2) configuré pour contenir des informations d'identification relatives à l'environnement confiné (1) et à son volume ;
prévoir dans l'environnement confiné (1) une pluralité d'objets, chacun étant équipé d'un moyen de reconnaissance associé (5) contenant des informations d'identification de l'objet et des informations relatives au volume de l'objet associé ;
prévoir un appareil de désinfection (3) dans l'environnement confiné pour distribuer des agents de décontamination par voie aérienne ;
acquérir les informations d'identification relatives à l'environnement confiné (1) à partir du dispositif d'identification environnementale (2), acquérir des informations à partir dudit moyen de reconnaissance (5) associé aux objets, acquérir des informations relatives à un agent de décontamination (10) à distribuer dans l'environnement confiné, et traiter lesdites informations acquises au moyen d'organes de commande et de traitement (17, 19) ;
calculer un volume d'air contenu dans l'environnement confiné (1) en soustrayant du volume de l'environnement confiné le volume occupé par les objets qui y sont contenus ;
calculer un temps de distribution en fonction du type d'agent de décontamination (10) distribué et du volume d'air contenu dans l'environnement confiné ;
distribuer ledit agent de décontamination (10) dans l'air au moyen d'un élément diffuseur (9) de l'appareil de désinfection (3) pendant le temps de distribution calculé par les organes de commande et de traitement (17, 19) sur la base des informations acquises ;
détecter la concentration de l'agent de décontamination par voie aérienne (10) dans l'environnement confiné (1) ; et
fournir des informations de certification, en fonction de la concentration détectée de l'agent de décontamination, certifiant que les surfaces de l'environnement confiné et les objets qui y sont contenus et associés aux moyens de reconnaissance (5) ont été désinfectés pendant l'étape de distribution de l'agent de décontamination.

2. Procédé selon la revendication 1, dans lequel la concentration de l'agent de décontamination par voie aérienne dans l'environnement confiné (1) est surveillée pendant la distribution dudit agent.

3. Procédé selon les revendications 1 ou 2, dans lequel la distribution de l'agent de décontamination (10) est contrôlée de manière à ce que la concentration dudit agent par voie aérienne (10) dans l'environnement confiné (1) ne descende pas en dessous d'une valeur de concentration prédéterminée pendant une période prédéterminée (26).

4. Procédé selon l'une quelconque des revendications précédentes, dans lequel la distribution de l'agent de décontamination (10) est prolongée si, pendant l'étape de distribution, la concentration détectée de l'agent de décontamination aéro-dispersé est inférieure à une valeur de concentration prédéterminée.

5. Procédé selon l'une quelconque des revendications précédentes, dans lequel la distribution de l'agent de décontamination (10) est relancée si, après expiration dudit temps de distribution calculé par les organes de commande et de traitement (17, 19) et l'arrêt de la distribution, la concentration détectée de l'agent de décontamination par voie aérienne est restée inférieure à une valeur de concentration prédéterminée pendant l'étape de distribution précédente.

6. Système de réalisation, de suivi, de surveillance et de contrôle de la désinfection par voie aérienne des surfaces d'un environnement confiné (1) et des objets qui y sont contenus, ledit système comprenant :
au moins un dispositif d'identification environnementale (2), situé dans un environnement confiné (1), ledit dispositif d'identification étant configuré pour contenir et fournir des informations d'identification relatives à l'environnement confiné (1) et des informations relatives au volume de l'environnement confiné (1) ;
au moins un dispositif d'identification (16) de l'agent de décontamination (10) configuré pour contenir et fournir des informations d'identification relatives à l'agent de décontamination (10) ;
une pluralité de moyens de reconnaissance (5), chacun étant associé à un objet situé dans l'environnement confiné (1), chacun desdits moyens de reconnaissance (5) contenant des informations d'identification relatives à l'objet associé et des informations relatives au volume de l'objet associé ;
un appareil de désinfection (3) équipé d'un diffuseur (9) pour la distribution par voie aérienne d'agents de décontamination (10) ;
des premiers moyens capteurs d'identification (4) pour acquérir les informations contenues dans les moyens de reconnaissance (5) associés aux objets situés à l'intérieur de l'environnement confiné ; des seconds moyens capteurs (8) adaptés pour acquérir des informations d'identification relatives à l'environnement confiné (1) à partir du dispositif d'identification environnementale (2), et adaptés pour acquérir des informations d'identification relatives à l'agent de décontamination (10) à distribuer dans l'environnement confiné (1) ;
un capteur de détection (11) adapté pour détecter la concentration de l'agent de décontamination (10) par voie aérienne à l'intérieur de l'environnement confiné (1) ;
des organes de commande et de traitement (17, 19) pour stocker et traiter les informations acquises par lesdits capteurs et fournir des informations de certification, en fonction de la concentration détectée de l'agent de décontamination, sur la conformité ou non-conformité de la désinfection des surfaces de l'environnement confiné et des objets qui y sont contenus pendant l'étape de distribution de l'agent de décontamination.

7. Système selon la revendication 6, dans lequel lesdits premiers et seconds moyens capteurs comprennent des capteurs configurés pour lire des étiquettes au moyen de la technologie RFID ou de code QR.

8. Système selon la revendication 6 ou 7, dans lequel lesdits seconds moyens capteurs (8) comprennent un transpondeur.

9. Système selon l'une quelconque des revendications 6 à 8, dans lequel l'appareil de désinfection (3) comprend : lesdits premiers (4) et seconds (8) moyens capteurs, le capteur de détection (11) de la concentration de l'agent de décontamination, les organes de commande et de traitement (17, 19).
